# EUROPEAN PATENT SPECIFICATION

(11) **EP 0 650 699 B1**
(45) Date of publication and mention of the grant of the patent: **04.03.1998**
(21) Application number: 94308093.7
(22) Date of filing: 02.11.1994
(51) Int. Cl.: A61B 17/06

(54) **Surgical needle tip/cutting edge protection**
Schutzvorrichtung für die Einstichspitze/Schneidkante einer chirurgischen Nadel
Protection pour la pointe ou le tranchant d'une aiguille chirurgicale

(30) Priority: 03.11.1993 US 147229
(43) Date of publication of application: 03.05.1995
(73) Proprietor: ETHICON, INC., Somerville, N J 08876 (US)
(72) Inventor: Brennan, Karl W., Somerset, NJ 08873 (US)
(74) Representative: Fisher, Adrian John

(56) References cited:
- EP-A- 0 500 229
- US-A- 5 089 001

## Description

### Background of the Invention

The present invention relates to surgical needles.

Surgical needles have been known for some time and such needles with sutures attached are commonly used in various types of surgical procedures. The surgical needles are usually made from stainless steel and have a blunt end to which a suture is attached. The blunt end may be drilled or have a channel placed therein and the suture is usually swaged or crimped in the drilled hole or channel. In order to reduce trauma in placing sutures in tissue, the suture itself generally has a diameter less than the diameter of the needle. Also, the point and cutting edge of the needle should be made as sharp as possible so as to require as little force as possible to penetrate the tissue and place the suture and consequently to cause the least amount of trauma to the sutured area.

Surgical needles are straight or curved, that is, they have the shape of some part of a circle. This may be from a quarter of a circle to five-eighths of a circle. At one end of the needle is the point or the extreme tip of the needle. The section from that point to the maximum width of the needle is termed the "blade" of the needle. In cutting needles, the blade includes the cutting edge from the point towards the blunt end. Behind the cutting edge is the body portion of the needle; that is, the area of the needle to be grasped by an appropriate needle holder. At the blunt end of the needle is the swage or the portion of the needle into which the suture is inserted and affixed. In placing the needle, the needle is grasped by a suitable needle holder which usually is a forceps type instrument that grasps the needle at the body portion with sufficient force to insure that the needle will not move or turn in the instrument when the needle is being placed by the surgeon.

Use of a needle can exert stressing forces on the needle, since the force used to drive the needle into and through tissue (e.g., a blood vessel, the cornea of the eye, and the like) needs to be sufficient to drive the needle and overcome frictional drag through the tissue. If the frictional drag is excessive, then applying this force along the shaft of the needle from the point at which the needle is held risks causing the needle to flex, which is undesirable as it causes loss of control of the needle.

The cutting edge and the point of the needle should also be as sharp as possible. The harder the needle the sharper it can be made. The sharper the needle, the less force required to make the initial penetration and the less the drag by the needle body during the remainder of the passage of the needle through tissue. However, the sharp needle tip poses a risk of puncture wounds to anyone handling it. Also, the sharp tip can become snagged on other material during the handling of needles in bulk associated with the manufacture and packaging of the needles. The tip itself is then also vulnerable to damage from inadvertent contact with other surfaces.

In general, surgical needles of the type described have been made of various stainless steels. Exemplary of such steels are AISI Type 420 stainless steel and ASTM 45500 stainless steel. The assignee of the present invention also markets needles under the name ETHALLOY^{(RTM)}, and has disclosed stainless steel compositions useful in making surgical needles in its U.S. Patent No. 5,000,912.

There is thus a need for surgical needles which permit the realization of the advantages described herein without the attendant risks described above.

### Summary of the Invention

In one aspect, the present invention comprises a surgical suture needle comprising a metal needle body ending in a tip adapted for piercing tissue, said needle body further comprising a protective polymeric film or polymeric cap encasing said tip the film or cap having been applied by dipping, wherein said film or cap is adherent to said tip and is completely removable therefrom manually.

In another aspect, the present invention comprises such a surgical suture needle wherein the needle has adhered thereto a layer of silicone polymer, and said film or cap encasing the tip is adherent to said layer and is completely removable therefrom manually.

### Brief Description of the Drawings

Fig. 1 is a side view of a needle having a protective film on the tip.

Fig. 2 is a side view of a needle having a protective cap on the tip.

### Detailed Description of the Invention

In composition, the sterile surgical needles of the present invention can be formed from any stainless steel or other alloy from which needles are or can be made. Examples include ASTM 45500, ASTM 42000 and ASTM 42020 stainless steel, and Type 420 stainless steel.

The needle will have a diameter effective to permit satisfactory usage in fine surgery. Typically, the diameter will be less than about 1.5mm (60 mils (thousandths of an inch)), preferably less than about 0.4mm (15 mils), down to about 0.025mm (1 mil), and preferably about 0.036 to about 0.3mm (1.4 to about 12 mils). It will be recognized that the needle can have the conventional circular cross-section, and that the needle may instead be of non-circular cross-sectional shape such as triangular; trapezoidal; rectangular; hexagonal; elliptical; or rectangular wherein the opposed shorter ends of the rectangle are rounded into semicircles. By "diameter" herein is meant the square root of (4A/π) where A is the cross-sectional area. The needle can be provided with a "ribbon" shape, or a rectangular or "I-beam" shape, or with a cross-section which smoothly undergoes transition from the point to a circular cross-section, to a rectangular cross-section having rounded and then sharper corners, as described in U.S. Patent No. 4,799,484.

The point can be a conventional tapered point. It can instead be a blunt point such as that described in U.S. Patent No. 5,123,910, a point having converging cutting edges as described in U.S. Patent No. 4,513,747, or any other point effective to promote smooth penetration into and passage through the tissue. The manner in which the needle is provided with the point of choice is quite familiar to the needle manufacturer.

The needle can be straight but preferably is curved through a radius of curvature which need not be constant but is preferably constant. Thus, more preferred shapes of the needles of the present invention comprise sections of a circle, such as a quarter circle, three-eighths circle, half circle, or five-eighths of a circle. As indicated above, the needle can comprise means at or near the blunt end furthest from the point, for securing a suture to the end of the needle. That means may comprise, for instance, a slot or channel in the outer surface of the needle or an opening in the end of the needle which is then swaged to hold the end of the suture.

The surgical needles of the present invention are characterized by a tensile yield strength generally on the order of about 1035 MN/m² (150,000 psi) or higher. A high tensile yield strength is useful as it indicates the ability of the needles of the present invention to withstand potentially deforming stresses during use, without suffering permanent deformation.

The needles of the present invention are also characterized by a tensile modulus of elasticity generally on the order of about 69 GN/m² (10⁷ psi) or higher. The high tensile modulus of elasticity is desirable in that it reflects higher stiffness and the ability of the needles of the present invention to withstand potentially deforming stresses during use by retaining their shape, without undue flexing. The tensile yield strength and tensile modulus of elasticity can be measured in accordance with the technique described in Bendel, L. and Trozzo, L., "Tensile and Bend Relationships of Several Surgical Needle Materials", J. of Applied Biomaterials, Vol. 4, pp. 161-167 (1993).

The needles of the present invention can be fabricated by processing the starting metal or alloy to normal mill standards to a size about 50% in area larger than the desired final size, annealing, and cold drawing to the finished size. The resulting wire is then cleaned free of surface oxides and other surface contaminants. Wire suitable as a starting material for making the needles of this invention is commercially available.

Following the final drawing to the final desired diameter, one end of the needle is given a point having the desired shape, the point being provided by any conventional technique such as grinding. Alternatively, the needle can be provided with a hardened point and cutting edge by exposing it to a laser beam or electron beam, as taught in U.S. Patent No. 4,660,559. The opposite end of the needle is given an opening in its end, or other means by which the end of a suture can be attached to the needle by swaging or the like.

The needle is then given its desired curvature, typically by rolling around a mandrel of the desired radius of curvature.

The tip of the needle is provided with a protective polymeric film or cap. The protective film can be on the order of a few mils thick where 1 mil is 0.0254mm, or it can take the form of a thicker cap or droplet of polymer. This protective film or cap must be thick enough so that it does not present a sharp tip which can snag on or puncture other material. Thus, it can take the shape of a rough globule, or it can more resemble the tapered tip of the needle itself although in a much softer and more rounded form, the object being that if the needle thus encased contacts another surface, or contacts the finger of the person handling it, the thus encased tip has a reduced risk of snagging or puncturing a surface with which it comes into contact.

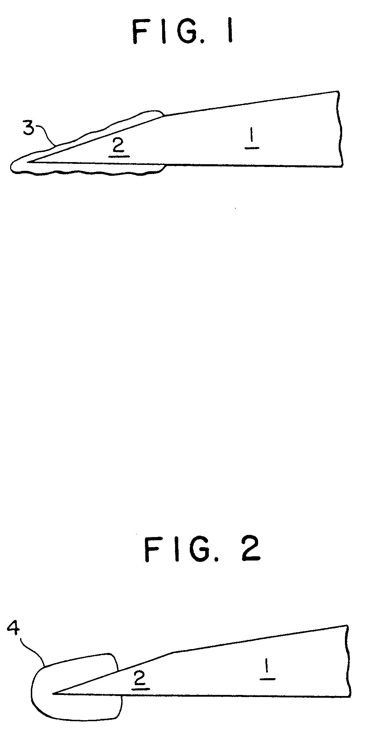

As seen in Fig. 1, the needle body 1 has a tip 2 which is encased in protective film 3. In Fig. 2, the protective cap 4 provides the same function. The exact shape and thickness of film 3 and cap 4 are not critical so long as it fulfills the functions of protecting the tip from damage upon contacting another surface, and preventing the tip from snagging on or puncturing other materials or the skin of a person handling the needle.

The material from which the protective film or cap is made is preferably polymeric and compatible with any silicone coating which may be present on the needle as described herein. Furthermore, the material is sufficiently adherent to the surface of the needle and the tip, or to any silicone coating present therein, so that the film or cap will not be dislodged from the needle when subjected to the normal handling to which the needle is subjected. On the other hand, the material from which the film or cap is made must be capable of being completely removed manually, that is, removed by manipulation by human fingers, when it is desired to remove the protective film or cap so that the needle can be used in surgery.

Exemplary materials from which such a protective film or cap can be made are any of the range of ethylene/vinyl acetate copolymers presently commercially available which are normally solid at ambient conditions. Examples of suitable ethylene/vinyl acetate copolymers abound in the commercial polymer field; a preferred example is known as "Elvax 210"^{(RTM)}, available from DuPont. Preferably, the ethylene vinyl acetate copolymer has a melting temperature of about 70°C to 90°C (160°F to about 200°F).

To apply the protective film or cap to the needle tip, one needs simply to melt a small quantity of the polymeric material to be used, and dip the tip of the needle into the melt. When a sufficiently thick layer has been supplied so as to provide the desired protection to the tip, using repeated dippings into the molten material if necessary, the needle is removed from the melt and the deposited material on the tip of the needle is air cooled whereby it is allowed to solidify.

Preferably, the film or cap is applied automatically by suspending needles vertically in a moving conveying system in which the needle tips are carried to the container holding the molten polymer, dipped therein for a predetermined period of time, withdrawn from the molten polymer, and conveyed to the next station in the sequence of steps whose final product is a sterile, packaged needle.

The needle is then packaged and the needle, suture and package are sterilized, again in accordance with conventional techniques. Thus, the polymer used as the film or cap needs to be inert to sterilization procedures employed. Conventional sterilization procedures include contact with ethylene oxide in a carrier gas, or irradiation with gamma radiation. Ethylene vinyl acetate copolymer satisfies this need.

That copolymer is also preferred because it is rather flexible in use, so that the film or cap is not brittle, or hard, or too rigid to remove.

The needles of the present invention can also be provided with a coating, of a polymeric silicone compound, also known herein as a polysiloxane coating. This coating preferably extends over the entirety of the surface of the needle body. The coating serves as a sort of lubricant to reduce frictional resistance of the tissue to the needle's piercing and passing into and through the tissue. The coating needs to be solid on the needle, and adherent to the needle material.

Suitable materials for the coating can be characterized as silicone polymers having a repeating unit of the formula (-Si-O-) wherein each Si atom carries two substituents, the polymer being capped at each end with a Si atom substituted with three substituents. Typically the substituents on the end-capping Si atoms are lower alkyl, preferably methyl. The substituents on the Si atoms of the repeating units can be hydrogen, lower alkyl (by which term is meant herein straight or branched alkyl containing one to six carbon atoms, preferably methyl), straight or branched alkylene groups containing two to six carbon atoms, alkoxy groups, alkylene oxy groups, phenyl, and combinations thereof. The silicone polymer is preferably hydrophobic. A preferred example is any dimethicone (that is, silicones substituted throughout with methyl groups) that is solid at temperatures up to about 50°C (120°F).

The silicone polymer preferably is of very high purity, suitable for use in surgical applications without risk that it or contaminants present in it might migrate out of the coating onto the adjacent tissue during use of the needle. Purities of at least 99.999 wt.% are therefore desired.

The silicone polymer coating can be provided to the needle by first putting the polymer in liquid form, preferably by forming a solution of the polymer in a suitable liquid solvent such as heptane. Then, the polymer is applied to the needle by spraying, dipping or otherwise coating the needle with a layer of the polymer. The needle is then air-dried under conditions that protect sterility and allow any solvent to evaporate. Preferably, the silicone coating can be more surely fixed to the needle by exposing the needle to mild heat following the application of the silicone polymer to the needle.

In use, a suture is attached to the blunt unpointed end of the needle. Then, the needle can be manipulated by hand or preferably can be gripped in an appropriate needle holder and then used in the desired surgical procedure.

## Claims

1. A surgical suture needle comprising a metal needle body (1) ending in a tip (2) adapted for piercing tissue, said needle body (1) further comprising a protective polymeric film (3) or polymeric cap (4) encasing said tip (2), the film (3) or cap (4) having been applied by dipping, wherein said film (3) or cap (4) is adherent to said tip (2) and is completely removable therefrom manually.

2. A surgical suture needle in accordance with claim 1 wherein said polymeric film (3) or polymeric cap (4) is made of ethylene vinyl acetate copolymer.

3. A surgical suture needle in accordance with claim 2 wherein said ethylene vinyl acetate copolymer has a melting temperature of about 70°C to 90°C (160°F to 200°F).

4. A surgical suture needle comprising a metal needle body (1) ending in a tip (2) adapted for piercing tissue, said needle body (1) having thereon an adherent layer of solid silicone polymer, and further comprising a protective polymeric film (3) or polymeric cap (4) encasing said tip (2), the film (3) or cap (4) having been applied by dipping, wherein said film (3) or cap (4) is adherent to said layer and is completely removable therefrom manually.

5. A surgical suture needle in accordance with claim 4 wherein said polymeric film (3) or polymeric cap (4) is made of ethylene vinyl acetate copolymer.

6. A surgical suture needle in accordance with claim 5 wherein said ethylene vinyl acetate copolymer has a melting temperature of about 70°C to 90°C (160°F to 200°F).

## Patentansprüche

1. Chirurgische Nähnadel, umfassend einen metallischen Nadelkörper (1), der in einem zum Einstechen in Gewebe geeigneten Spitze (2) endet, wobei der Nadelkörper (1) ferner einen schützenden polymeren Film (3) oder eine schützende polymere Kappe (4) umfaßt, die die Spitze (2) umhüllen, wobei der Film (3) oder die Kappe (4) durch eine Tauchbehandlung aufgebracht worden sind und wobei der Film (3) oder die Kappe (4) an der Spitze (2) haften und davon auf manuellem Wege vollständig entfernbar sind.

2. Chirurgische Nähnadel nach Anspruch 1, wobei der polymere Film (3) oder die polymere Kappe (4) aus einem Ethylen-Vinylacetat-Copolymeren gebildet sind.

3. Chirurgische Nähnadel nach Anspruch 2, wobei das Ethylen-Vinylacetat-Copolymere eine Schmelztemperatur von etwa 70 bis 90°C (160 bis 200°F) aufweist.

4. Chirurgische Nähnadel, umfassend einen metallischen Nadelkörper (1), der in einer zum Einstechen in Gewebe geeigneten Spitze (2) endet, wobei am Nadelkörper (1) eine Schicht aus einem festen Silicon-Polymeren haftet, und ferner umfassend einen schützenden polymeren Film (3) oder eine schützende polymere Kappe (4), die die Spitze (2) umhüllen, wobei der Film (3) oder die Kappe (4) durch eine Tauchbehandlung aufgebracht worden sind und wobei der Film (3) oder die Kappe (4) an der Schicht haften und davon auf manuellem Wege vollständig entfernbar sind.

5. Chirurgische Nähnadel nach Anspruch 4, wobei der polymere Film (3) oder die polymere Kappe (4) aus einem Ethylen-Vinylacetat-Copolymeren gebildet sind.

6. Chirurgische Nähnadel nach Anspruch 5, wobei das Ethylen-Vinylacetat-Copolymere eine Schmelztemperatur von etwa 70 bis 90°C (160 bis 200°F) aufweist.

## Revendications

1. Aiguille chirurgicale pour suture comportant un corps métallique d'aiguille (1) se terminant en une pointe (2) conçue pour percer les tissus, ledit corps d'aiguille (1) comportant en outre un film polymère protecteur (3) ou un capuchon polymère (4) enveloppant ladite pointe (2), le film (3) ou le capuchon (4) ayant été appliqués au trempé, dans laquelle ledit film (3) ou ledit capuchon (4) adhère à ladite pointe (2) et peut en être complètement extrait manuellement.

2. Aiguille chirurgicale pour suture selon la revendication 1, dans laquelle ledit film polymère (3) ou ledit capuchon polymère (4) est fait d'un copolymère éthylène-acétate de vinyle.

3. Aiguille chirurgicale pour suture selon la revendication 2, dans laquelle ledit copolymère éthylène-acétate de vinyle a une température de fusion allant d'environ 70°C à 90°C (160°F à 200°F).

4. Aiguille chirurgicale pour suture comportant un corps métallique d'aiguille (1) se terminant en une pointe (2) conçue pour percer les tissus, ledit corps d'aiguille (1) présentant sur lui une couche adhérente d'un polymère de silicone solide et comportant en outre un film polymère protecteur (3) ou un capuchon polymère (4) enveloppant ladite pointe (2), le film (3) ou le capuchon (4) ayant été appliqués au trempé, dans laquelle ledit film (3) ou ledit capuchon (4) adhère à ladite couche et peut en être complètement extrait manuellement.

5. Aiguille chirurgicale pour suture selon la revendication 4, dans laquelle ledit film polymère (3) ou ledit capuchon polymère (4) est fait d'un copolymère éthylène-acétate de vinyle.

6. Aiguille chirurgicale pour suture selon la revendication 5, dans laquelle ledit copolymère éthylène-acétate de vinyle a une température de fusion allant d'environ 70°C à 90°C (160°F à 200°F).
